# EUROPEAN PATENT APPLICATION

(11) **EP 4 814 479 A1**
(43) Date of publication of application: **30.09.2026**
(21) Application number: 24912043.7
(22) Date of filing: 08.11.2024
(51) Int. Cl.: G01N 21/65, C07K 1/16, C07K 16/00

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 25.12.2023 JP 2023218334
(71) Applicant: FUJIFILM Corporation, Tokyo 106-8620 (JP)
(72) Inventor: SUGITA, Yui, Ashigarakami-gun, Kanagawa 258-8577 (JP)
(74) Representative: Hoffmann Eitle
(86) International application number: PCT/JP2024/039828
(87) International publication number: WO 2025/142150

(57) **Abstract**

An information processing apparatus that predicts an amount of a fragment of a biological molecule based on spectral data obtained by measuring electromagnetic waves emitted from a suspension in which the biological molecule and the fragment of the biological molecule are dispersed as components in a liquid, the information processing apparatus including a processor, in which the processor is configured to use a prediction model in which at least one of generation processing or data input processing according to a difference occurring in a specific spectral region between spectral data of the biological molecule and spectral data of the fragment is executed, acquire target spectral data of a target suspension in which the amount of the fragment is unknown, and input the target spectral data to the prediction model to predict the amount of the fragment.

## Description

### BACKGROUND OF THE INVENTION

### Technical Field

The disclosed technology relates to an information processing apparatus, an operation method for an information processing apparatus, and an operation program of an information processing apparatus.

For example, a manufacturing process of a biopharmaceutical in which a biological molecule such as a protein such as a monoclonal antibody is used as an active ingredient is known. In such a manufacturing process, a suspension in which various components including the active ingredient are dispersed in a liquid is often produced. It is important to monitor an amount of a target component among the components in the suspension in order to lead a manufacturing process in progress to success. Examples of the target component include a fragment of a protein (hereinafter, simply referred to as a fragment), and examples of the amount of the target component include a content and a concentration of the fragment. Since the presence of the fragment affects the effectiveness and safety of the biopharmaceutical, it is particularly important to monitor the amount of the target component as the fragment.

As a technology for predicting the amount of the target component, the following technology is attracting attention for a reason that there is little concern about contamination. That is, the technology is a technology in which an electromagnetic wave such as Raman scattered light emitted from the suspension is measured, and spectral data such as Raman spectral data obtained by the measurement is input to a prediction model such as multivariate analysis or a machine learning model to cause the prediction model to predict the amount of the target component. For example, JP7326307B and JP2021-535739A describe that the fragment is used as the target component and the amount of the fragment is predicted by the prediction model.

Since the fragment is naturally derived from the protein, information on a molecular structure such as an amino acid sequence is not different from that of the protein. Therefore, in a suspension in which the protein and the fragment are mixed, in a case where the fragment is used as the target component as in JP7326307B and JP2021-535739A, there is a concern that prediction accuracy of the amount of the fragment may be low.

### SUMMARY

One embodiment according to the disclosed technology provides an information processing apparatus, an operation method for an information processing apparatus, and an operation program of an information processing apparatus capable of accurately predicting an amount of a fragment in a suspension in which a biological molecule and a fragment of the biological molecule are mixed.

The information processing apparatus according to the present disclosure is an information processing apparatus that predicts an amount of a fragment of a biological molecule based on spectral data obtained by measuring electromagnetic waves emitted from a suspension in which the biological molecule and the fragment of the biological molecule are dispersed as components in a liquid, the information processing apparatus including a processor, in which the processor is configured to use a prediction model in which at least one of generation processing or data input processing according to a difference occurring in a specific spectral region between spectral data of the biological molecule and spectral data of the fragment is executed, acquire target spectral data of a target suspension in which the amount of the fragment is unknown, and input the target spectral data to the prediction model to predict the amount of the fragment.

It is preferable that the data input processing is processing for selectively inputting data of the specific spectral region among the target spectral data to the prediction model.

It is preferable that the prediction model is generated by using calibration data including reference spectral data of a reference suspension in which the amount of the fragment is known, and the generation processing is processing for including spectral data of a standard sample of the fragment artificially generated from the biological molecule in the calibration data as the reference spectral data.

It is preferable that the data input processing is processing for selectively inputting data of the specific spectral region among the reference spectral data to the prediction model.

It is preferable that the biological molecule is a protein, and the electromagnetic waves are Raman scattered light.

It is preferable that the specific spectral region is a region attributed to at least one of a disulfide bond, an aromatic amino acid, a carbon-carbon bond, a carbon-nitrogen bond, or a carbon-oxygen bond of the protein.

It is preferable that the specific spectral region is at least one of a region having a wave number of 470 cm⁻¹ to 550 cm⁻¹, a region having a wave number of 860 cm⁻¹ to 900 cm⁻¹, or a region having a wave number of 1060 cm⁻¹ to 1120 cm⁻¹.

It is preferable that the protein is an antibody.

It is preferable that the fragment is at least one of a heavy chain, a light chain, a fragment antigen-binding region, and a fragment crystallizable region of the antibody.

It is preferable that the target suspension is a purified liquid obtained by passing a culture supernatant liquid of a cell that produces the antibody through a chromatography device.

It is preferable that the specific spectral region is a region identified by comparing the spectral data of the biological molecule with spectral data of a standard sample of the fragment artificially generated from the biological molecule.

It is preferable that the prediction model is a machine learning model.

It is preferable that a concentration of the fragment in the target suspension is 0.1 g/L to 5.0 g/L.

An operation method for an information processing apparatus according to the present disclosure is an operation method for an information processing apparatus that predicts an amount of a fragment of a biological molecule based on spectral data obtained by measuring electromagnetic waves emitted from a suspension in which the biological molecule and the fragment of the biological molecule are dispersed as components in a liquid, the operation method for an information processing apparatus including using a prediction model in which at least one of generation processing or data input processing according to a difference occurring in a specific spectral region between spectral data of the biological molecule and spectral data of the fragment is executed, acquiring target spectral data of a target suspension in which the amount of the fragment is unknown, and inputting the target spectral data to the prediction model to predict the amount of the fragment.

An operation program of an information processing apparatus according to the present disclosure is an operation program of information processing apparatus that predicts an amount of a fragment of a biological molecule based on spectral data obtained by measuring electromagnetic waves emitted from a suspension in which the biological molecule and the fragment of the biological molecule are dispersed as components in a liquid, the operation program causing a computer to execute a process including using a prediction model in which at least one of generation processing or data input processing according to a difference occurring in a specific spectral region between spectral data of the biological molecule and spectral data of the fragment is executed, acquiring target spectral data of a target suspension in which the amount of the fragment is unknown, and inputting the target spectral data to the prediction model to predict the amount of the fragment.

According to the disclosed technology, it is possible to provide an information processing apparatus, an operation method for an information processing apparatus, and an operation program of an information processing apparatus capable of accurately predicting an amount of a fragment in a suspension in which a biological molecule and a fragment of the biological molecule are mixed.

### BRIEF DESCRIPTION OF THE DRAWINGS

FIG. 1 is a diagram showing a measurement system, a culture unit, a purification unit, and an information processing apparatus.
FIG. 2 is a diagram showing a basic structure of an antibody.
FIG. 3 is a diagram showing excitation light and Raman scattered light.
FIG. 4 is a diagram showing Raman spectral data.
FIG. 5 is a block diagram of a computer constituting the information processing apparatus.
FIG. 6 is a block diagram showing a processing unit of a CPU of the information processing apparatus.
FIG. 7 is a diagram showing processing for a learning unit.
FIG. 8 is a diagram showing a processing for a prediction unit.
FIG. 9 is a flowchart showing a flow from generation of a fragment standard sample to measurement of fragment standard sample Raman spectral data.
FIG. 10 is a diagram showing a specific spectral region identified by comparison between antibody Raman spectral data and fragment standard sample Raman spectral data.
FIG. 11 is a diagram showing generation processing for generating a plurality of fragment standard sample solutions having different contents and storing fragment standard sample Raman spectral data and a fragment content as training data.
FIG. 12 is a diagram showing a data input screen.
FIG. 13 is a diagram showing a prediction result display screen.
FIG. 14 is a flowchart showing a flow of the generation processing.
FIG. 15 is a flowchart showing a flow of processing in a learning phase of a prediction model.
FIG. 16 is a flowchart showing a flow of processing for the information processing apparatus.
FIG. 17 is a graph showing a temporal transition of a fragment content by the prediction model in Example 1 and a temporal transition of a fragment content by offline analysis.
FIG. 18 is a table showing evaluation results of prediction accuracy of a fragment content in Examples and Comparative Example.

### DETAILED DESCRIPTION

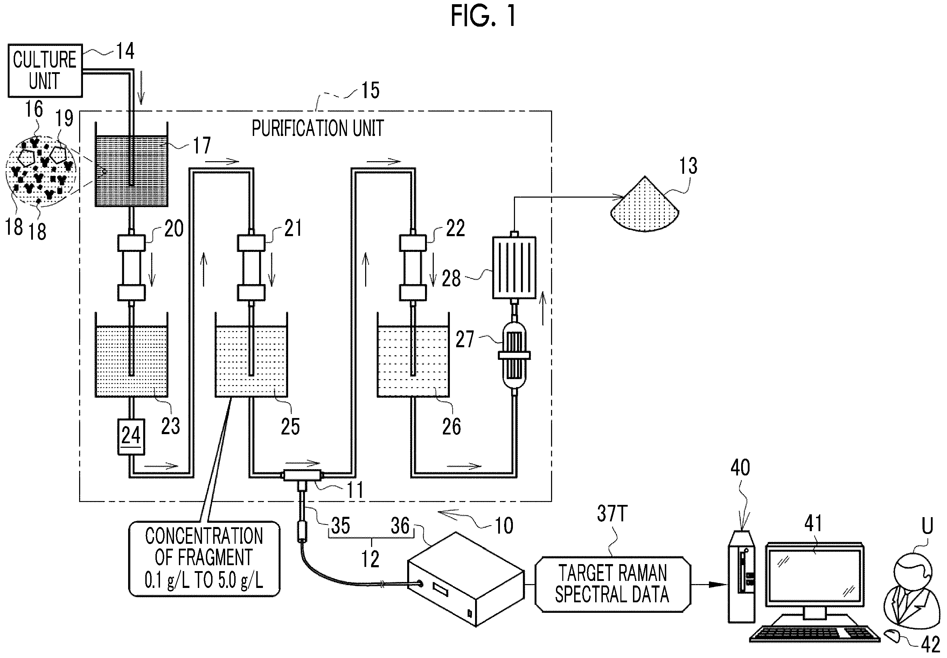

As shown in FIG. 1 as an example, a measurement system 10 comprises a flow cell 11 and a Raman spectrometer 12. The measurement system 10 is incorporated, for example, in a purification unit 15 following a culture unit 14 in a manufacturing system of an active pharmaceutical ingredient 13 of a biopharmaceutical.

The culture unit 14 has a culture tank and a cell removal filter. A cell culture solution (culture medium) is stored in the culture tank. Antibody-producing cells are seeded in the cell culture solution, and the antibody-producing cells are cultured in the cell culture solution. A culture method may be any of perfusion culture or fed-batch culture. The antibody-producing cells are, for example, cells established by incorporating an antibody gene into a host cell such as Chinese hamster ovary cells (CHO cells). The antibody-producing cells produce an immunoglobulin, that is, an antibody 16 in the process of culture. Therefore, the antibody 16 is present in the cell culture solution in addition to the antibody-producing cells. The antibody 16 is, for example, a monoclonal antibody, and serves as an active ingredient of the biopharmaceutical. The antibody 16 is an example of a "biological molecule", a "component", and a "protein" according to the technology of the present disclosure.

The cell removal filter captures the antibody-producing cells in the cell culture solution with a filter membrane by, for example, a tangential flow filtration (TFF) method or an alternating tangential flow filtration (ATF) method, and removes the antibody-producing cells from the cell culture solution. In addition, the cell removal filter allows the antibody 16 to permeate. Therefore, the cell culture solution output from the culture unit 14 to the purification unit 15 mainly contains the antibody 16. Hereinafter, the cell culture solution output from the culture unit 14 to the purification unit 15 is referred to as a culture supernatant liquid 17. The culture supernatant liquid 17 contains, in addition to the antibody 16, a cell-derived protein, a cell-derived deoxyribonucleic acid (DNA), a fragment 18 of the antibody 16, an aggregate 19 of the antibody 16, a virus, or the like. The cell-derived protein, the cell-derived DNA, the fragment 18, the aggregate 19, the virus, and the like are also examples of a "component" according to the technology of the present disclosure. In addition, the culture supernatant liquid 17 is an example of a "suspension" according to the technology of the present disclosure.

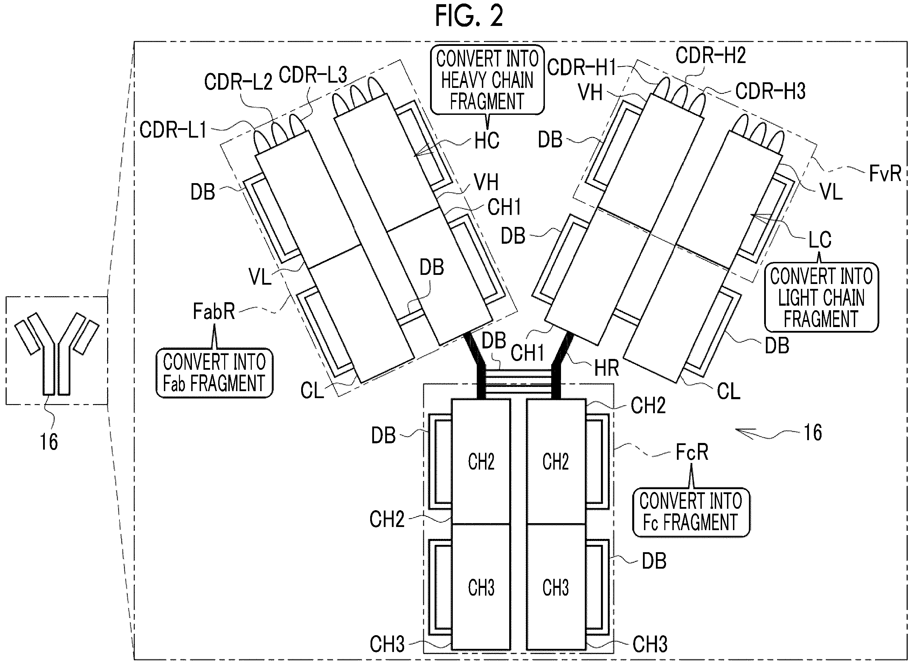

As illustrated in FIG. 2 as an example, the antibody 16 basically has four polypeptide chains, that is, two identical heavy chains HC and two identical light chains LC. The antibody 16 has a configuration in which the two heavy chains HC and the two light chains LC are bonded by a disulfide bond DB, and has a Y shape that is bilaterally symmetrical.

The heavy chain HC is composed of a variable domain VH (variable domain, heavy chain) and constant domains CH (constant domain, heavy chain) 1, CH2, and CH3. The light chain LC is composed of a variable domain VL (variable domain, light chain) and a constant domain CL (constant domain, light chain). The constant domains CH1 and CH2 of the heavy chain HC are connected by a hinge region HR. The variable domains VH and VL are collectively referred to as a variable region. The constant domains CH1 to CH3 and CL are collectively referred to as a constant region.

The variable domain VH includes three complementarity determining regions, heavy chain (CDR-H)1, CDR-H2, and CDR-H3. In addition, the variable domain VL also includes three complementarity determining regions, light chain (CDR-L) 1, CDR-L2, and CDR-L3. These complementarity determining regions CDR-H1 to CDR-H3 and CDR-L1 to CDR-L3 are bonding sites with an antigen and are also referred to as a hypervariable region.

A region composed of the variable domains VH and VL and the constant domains CH1 and CL is a fragment antigen-binding region (FabR). A region composed of the constant domains CH2 and CH3 and a part of the hinge region HR is a fragment crystallizable region (FcR). A region composed of the variable domains VH and VL is a fragment variable region (FvR).

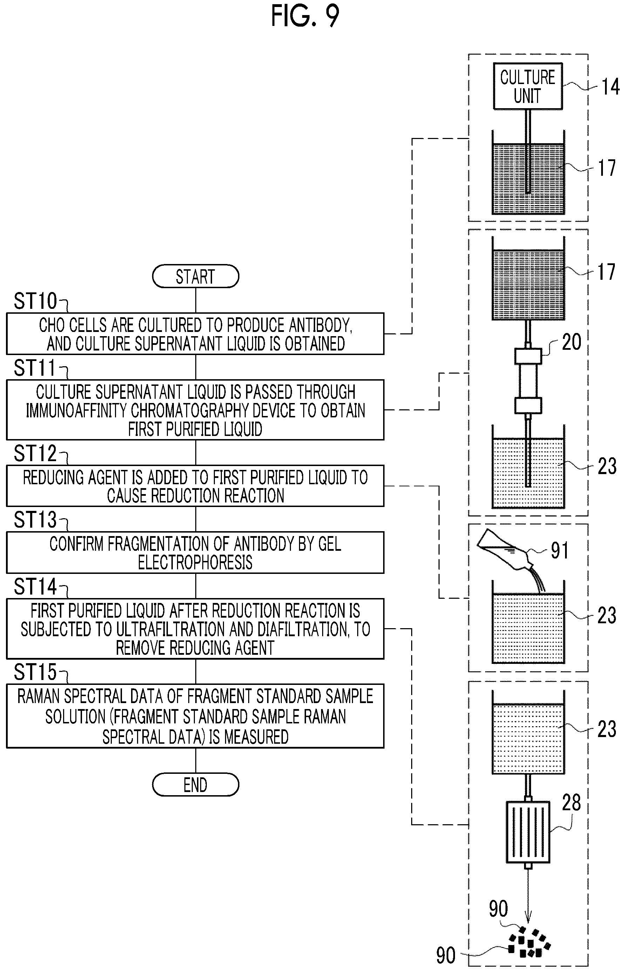

By causing an enzyme reaction or a reduction reaction, each portion of the antibody 16, such as the heavy chain HC, the light chain LC, the fragment antigen-binding region FabR, and the fragment crystallizable region FcR, is converted into a fragment 18 (a heavy chain fragment, a light chain fragment, an Fab fragment, and an Fc fragment). Both the enzyme reaction and the reduction reaction are reactions for fragmenting the antibody 16 by digesting or cleaving a specific disulfide bond DB. In the enzyme reaction, an enzyme such as papain acts as a catalyst. In the reduction reaction, a reducing agent 91 (see FIG. 9) such as tris(2-carboxyethyl)phosphine (TCEP) acts as a catalyst.

Returning to FIG. 1, the purification unit 15 performs a component separation treatment on the culture supernatant liquid 17 from the culture unit 14 in order by a plurality of chromatography devices to remove impurities and viruses in a stepwise manner and to increase the purity of the antibody 16 in the culture supernatant liquid 17 in a stepwise manner. The plurality of chromatography devices include, for example, an immunoaffinity chromatography device 20, a cation exchange chromatography device 21, and an anion exchange chromatography device 22 in order from the upstream side. The immunoaffinity chromatography device 20, the cation exchange chromatography device 21, and the anion exchange chromatography device 22 are examples of a "chromatography device" according to the disclosed technology. The chromatography device may include a size exclusion chromatography device, a hydrophobic interaction chromatography device, and the like.

The culture supernatant liquid 17 is introduced into the immunoaffinity chromatography device 20. The immunoaffinity chromatography device 20 extracts the antibody 16 from the culture supernatant liquid 17 by using a column in which a ligand such as a protein A having an affinity for the antibody 16 is immobilized on a carrier, and thereby generating a first purified liquid 23. The first purified liquid 23 is subjected to the virus inactivating treatment 24.

The first purified liquid 23 after the virus inactivation treatment 24 is introduced into the cation exchange chromatography device 21. The cation exchange chromatography device 21 extracts the antibody 16 from the first purified liquid 23 using a column in which a cation exchanger is used as a stationary phase, thereby producing a second purified liquid 25. The cation exchange chromatography device 21 separates and elutes the fragment 18, the antibody 16, and the aggregate 19 in this order by a method called gradient elution in which the salt concentration in the buffer solution for washing the column is temporally increased.

The second purified liquid 25 is introduced into the anion exchange chromatography device 22. The anion exchange chromatography device 22 extracts the antibody 16 from the second purified liquid 25 using a column in which an anion exchanger is used as a stationary phase, thereby producing a third purified liquid 26.

The third purified liquid 26 is passed through a filter 27 to remove the virus. Thereafter, the third purified solution 26 is subjected to a concentration/filtration treatment via ultrafiltration (UF) and diafiltration (DF) using the filter 28. The active pharmaceutical ingredient 13 of the biopharmaceutical is obtained from the third purified liquid 26 thus treated. It should be noted that a single pass tangential flow filtration (SPTFF) type filter may be provided in front of the immunoaffinity chromatography device 20.

As in the culture supernatant liquid 17, the antibody 16 and the fragment 18 thereof are mixed in the first purified liquid 23, the second purified liquid 25, and the third purified liquid 26. However, since the purity of the antibody 16 is increased stepwise by each of the chromatography devices 20 to 22, the antibody 16 occupies most of the content, and the fragment 18 is very small. For example, the concentration of the fragment 18 in the second purified liquid 25 is 0.1 g/L to 5.0 g/L (0.1 g/L or more and 5 g/L or less). The first purified liquid 23, the second purified liquid 25, and the third purified liquid 26 are examples of a "suspension" and a "purified liquid" according to the technology of the present disclosure.

The flow cell 11 is installed in a flow passage that connects the cation exchange chromatography device 21 and the anion exchange chromatography device 22. The second purified liquid 25 flowing from the cation exchange chromatography device 21 toward the anion exchange chromatography device 22 flows through the flow cell 11 at a preset flow rate.

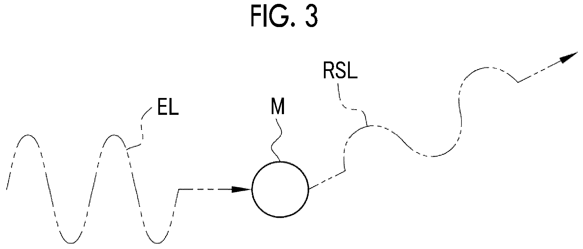

As shown in FIG. 3 as an example, the Raman spectrometer 12 is a device that evaluates a substance M by using the characteristics of Raman scattered light RSL. In a case in which the substance M is irradiated with excitation light EL, the excitation light EL interacts with the substance M to generate the Raman scattered light RSL having a wavelength different from the excitation light EL. A wavelength difference between the excitation light EL and the Raman scattered light RSL corresponds to the energy of the molecular vibration of the substance M. For this reason, it is possible to obtain the Raman scattered light RSL having different wave numbers between the substances M having different molecular structures. The Raman scattered light RSL is an example of an "electromagnetic wave" according to the technology of the present disclosure. It should be noted that it is preferable that the Raman scattered light RSL is a Stokes ray out of the Stokes ray and an anti-Stokes ray.

Returning to FIG. 1, the Raman spectrometer 12 is configured by a sensor unit 35 and an analyzer 36. The sensor unit 35 is connected to the flow cell 11. The sensor unit 35 emits the excitation light EL from a distal end thereof. The excitation light EL is irradiated to the second purified liquid 25 flowing in the flow cell 11. The Raman scattered light RSL is generated by the interaction between the excitation light EL and the components such as the antibody 16 and the fragment 18 in the second purified liquid 25. The sensor unit 35 receives the Raman scattered light RSL, and outputs the received Raman scattered light RSL to the analyzer 36.

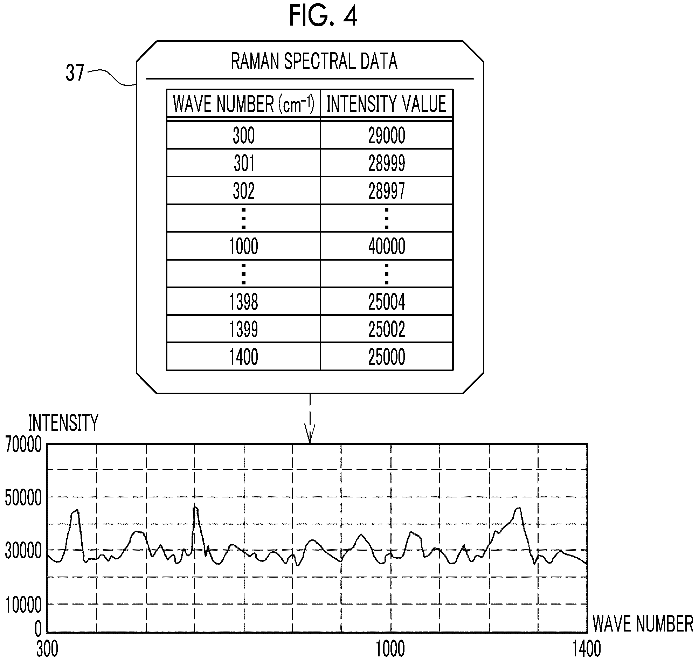

The analyzer 36 generates Raman scattered light RSL spectrum, that is, Raman spectral data 37 representing a Raman spectrum as shown in FIG. 4 as an example by decomposing the Raman scattered light RSL for each wave number and deriving an intensity value of the Raman scattered light RSL for each wave number. The Raman spectral data 37 is data in which the intensity value of the Raman scattered light RSL is registered for each wave number. In the present example, the Raman spectral data 37 is data in which intensity values of the Raman scattered light RSL in a range of wave numbers of 300 cm⁻¹ to 1400 cm⁻¹ are derived in increments of 1 cm⁻¹. In FIG. 4, a graph shown in a lower part of the Raman spectral data 37 is a graph in which the intensity values of the Raman spectral data 37 are plotted for each wave number and connected by a line. The Raman spectral data 37 is an example of "spectral data" according to the disclosed technology.

As described above, the measurement system 10 causes the second purified liquid 25 to flow into the flow cell 11. Then, the second purified liquid 25 flowing through the flow cell 11 is irradiated with the excitation light EL through the sensor unit 35, so that the Raman scattered light RSL of the components such as the antibody 16 and the fragment 18 in the second purified liquid 25 is measured, and the Raman spectral data 37 is obtained. That is, the second purified liquid 25 is an example of a "target suspension" according to the disclosed technology.

Returning to FIG. 1 again, the information processing apparatus 40 is connected to the Raman spectrometer 12 through a computer network such as a local area network (LAN). The Raman spectrometer 12 transmits the measured Raman spectral data 37 to the information processing apparatus 40. The Raman spectral data 37 transmitted from the Raman spectrometer 12 to the information processing apparatus 40 is data for predicting a content of the fragment 18 in the second purified liquid 25 (hereinafter, referred to as a fragment content). The fragment content is a ratio of an amount of the fragment 18 to an amount of the suspension including the fragment 18 as a component, here, the second purified liquid 25. For example, in a case where the amount of the second purified liquid 25 is 100 g and the amount of the fragment 18 in the second purified liquid 25 is 5 g, the fragment content is (5/100) × 100 = 5%. The fragment content is an example of an "amount of the fragment" according to the disclosed technology. In the following description, the Raman spectral data 37 transmitted from the Raman spectrometer 12 to the information processing apparatus 40 is referred to as target Raman spectral data 37T. The target Raman spectral data 37T is an example of "target spectral data" according to the disclosed technology.

The information processing apparatus 40 is, for example, a desktop personal computer, and comprises a display 41 that displays various screens and an input device 42, such as a keyboard, a mouse, a touch panel, and/or a microphone for audio input. The information processing apparatus 40 is installed in, for example, a pharmaceutical company that develops a biopharmaceutical or an institution that receives a business of developing a biopharmaceutical from a pharmaceutical company, that is, a contract research organization (CRO). The information processing apparatus 40 is operated by a user U involved in the development of a biopharmaceutical in a pharmaceutical company or a contract research organization (hereinafter, collectively referred to as a pharmaceutical facility).

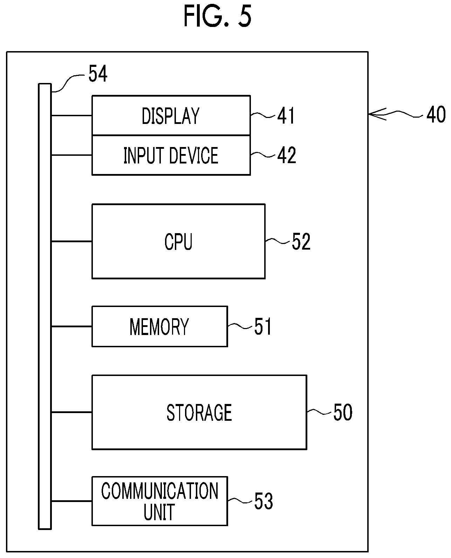

For example, as shown in FIG. 5, the computer constituting the information processing apparatus 40 comprises a storage 50, a memory 51, a central processing unit (CPU) 52, and a communication unit 53, in addition to the display 41 and the input device 42 described above. These components are connected to each other through a busline 54.

The storage 50 is a hard disk drive that is incorporated in the computer constituting the information processing apparatus 40 or connected to the computer through a cable or a network. Alternatively, the storage 50 is a disk array in which a plurality of hard disk drives are connected. The storage 50 stores, for example, a control program, such as an operating system, various application programs, and various types of data associated with these programs. Note that a solid-state drive may be used instead of the hard disk drive.

The memory 51 is a work memory used by the CPU 52 to execute processing. The CPU 52 loads the program stored in the storage 50 to the memory 51 and executes a process corresponding to the program. Therefore, the CPU 52 controls each unit of the computer comprehensively. The CPU 52 is an example of a "processor" according to the technology of the present disclosure. In addition, the memory 51 may be provided in the CPU 52. The communication unit 53 performs transmission control of various information with an external device, such as the Raman spectrometer 12.

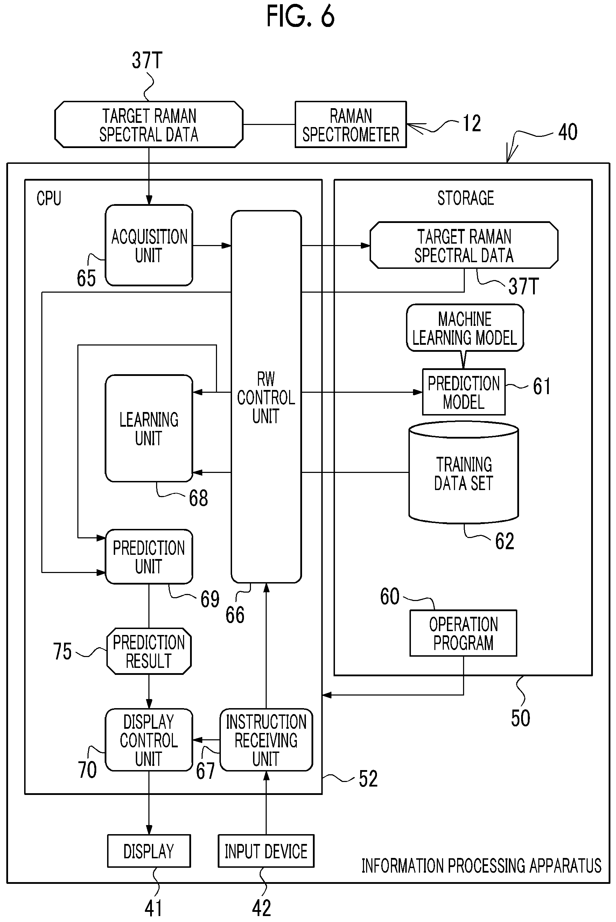

As shown in FIG. 6 as an example, an operation program 60 is stored in the storage 50. The operation program 60 is an application program for making a computer function as the information processing apparatus 40. That is, the operation program 60 is an example of an "operation program of an information processing apparatus" according to the technology of the present disclosure. In addition to the operation program 60, the storage 50 also stores a prediction model 61 and a training data set 62. The prediction model 61 is a machine learning model using an algorithm such as a decision tree, a gradient boosting decision tree, a naive Bayes, a random forest, a support vector machine, or a neural network.

In a case where the operation program 60 is started, the CPU 52 of the computer constituting the information processing apparatus 40 functions as an acquisition unit 65, a read/write (hereinafter, abbreviated as RW) control unit 66, an instruction receiving unit 67, a learning unit 68, a prediction unit 69, and a display control unit 70 in cooperation with the memory 51 and the like.

The acquisition unit 65 acquires the target Raman spectral data 37T from the Raman spectrometer 12. The acquisition unit 65 outputs the target Raman spectral data 37T to the RW control unit 66.

The RW control unit 66 controls the storage of various types of data into the storage 50 and the readout of various types of data stored in the storage 50. The RW control unit 66 stores the target Raman spectral data 37T from the acquisition unit 65 in the storage 50. In FIG. 6, the target Raman spectral data 37T is illustrated as being stored in the storage 50 only once, but in reality, a plurality of pieces of the target Raman spectral data 37T are stored in the storage 50.

The RW control unit 66 reads out the prediction model 61 and the training data set 62 from the storage 50, and outputs the readout prediction model 61 and training data set 62 to the learning unit 68. In addition, the RW control unit 66 reads out the target Raman spectral data 37T and the trained prediction model 61 from the storage 50, and outputs the readout target Raman spectral data 37T and prediction model 61 to the prediction unit 69.

The instruction receiving unit 67 receives various operation instructions input by the user U via the input device 42. The operation instruction includes an input instruction of the target Raman spectral data 37T of the second purified liquid 25 for which the fragment content is to be predicted, a prediction instruction of the fragment content, and the like.

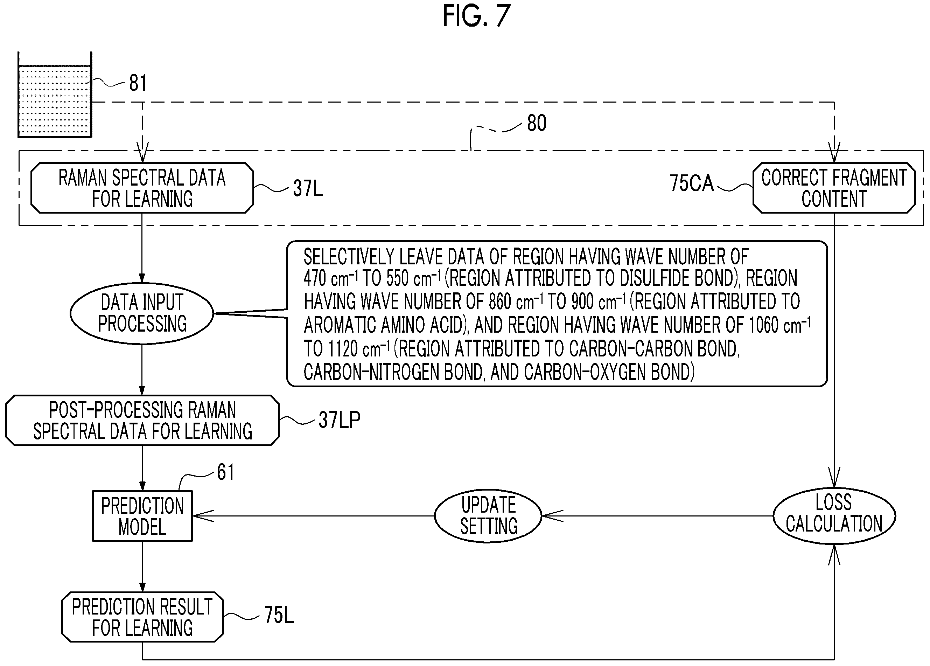

The learning unit 68 trains the prediction model 61 using a plurality of pieces of training data 80 (see FIG. 7) constituting the training data set 62. The learning unit 68 outputs the trained prediction model 61 to the RW control unit 66. The RW control unit 66 stores the trained prediction model 61 from the learning unit 68 in the storage 50.

The prediction unit 69 inputs the target Raman spectral data 37T to the prediction model 61 to cause the prediction model 61 to predict the fragment content, and outputs a prediction result 75 from the prediction model 61. The prediction unit 69 outputs the prediction result 75 to the display control unit 70.

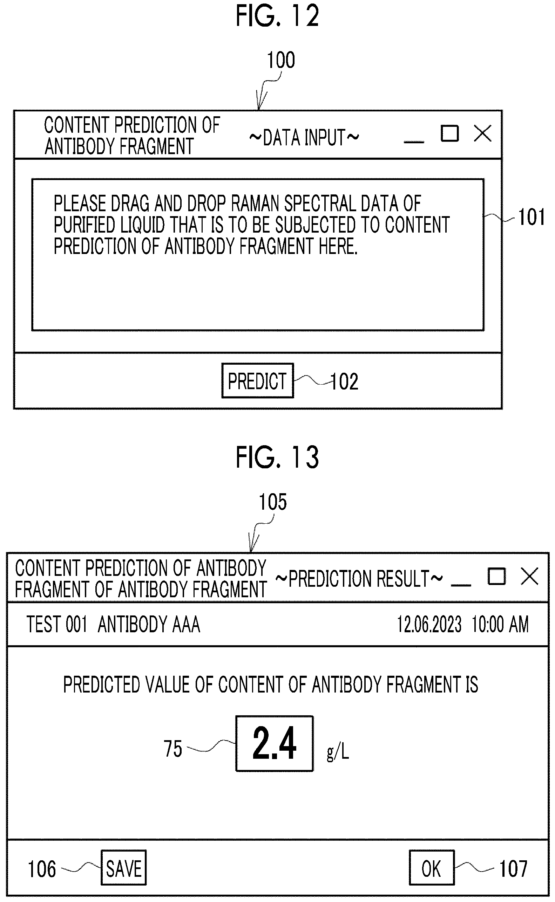

The display control unit 70 controls display of various screens on the display 41. For example, the display control unit 70 performs control of displaying, on the display 41, a data input screen 100 (see FIG. 12) for performing an input instruction of the target Raman spectral data 37T of the second purified liquid 25 for which the fragment content is to be predicted and a prediction instruction of the fragment content, a prediction result display screen 105 (see FIG. 13) for displaying the prediction result 75, and the like.

As shown in FIG. 7 as an example, the learning unit 68 uses training data (also referred to as teacher data or training data) 80 for training the prediction model 61. The training data 80 is a set of Raman spectral data for learning 37L and a correct fragment content 75CA. The Raman spectral data for learning 37L is obtained by measuring Raman scattered light RSL emitted from a reference suspension 81 with the Raman spectrometer 12. The reference suspension 81 is a suspension containing at least the fragment 18. The reference suspension 81 includes, for example, the second purified liquid 25 of which the fragment content is known, which is generated in the past. The correct fragment content 75CA is a fragment content calculated based on the amount of the fragment 18 in the reference suspension 81. The amount of the fragment 18 in the reference suspension 81 is obtained, for example, by introducing the reference suspension 81 into a high performance liquid chromatography (hereinafter, referred to as high performance liquid chromatography (HPLC)) apparatus and measuring the amount of the fragment 18 by a mass spectrometry function using an ultraviolet absorption method provided in the HPLC apparatus. The training data 80 is an example of "calibration data" according to the technology of the present disclosure. In addition, the Raman spectral data for learning 37L is an example of "reference spectral data" according to the technology of the present disclosure. As a method of quantifying the amount of the fragment 18, the HPLC and the ultraviolet absorption method described as an example are preferable, but the method is not limited thereto. For example, a gel electrophoresis such as polyacrylamide gel electrophoresis (sodium dodecyl sulfate-polyacrylamide gel electrophoresis (SDS-PAGE)) or a capillary electrophoresis may be used. In addition, an immunoturbidimetric method, a colorimetric method, a fluorescence method, or the like may be used.

The learning unit 68 performs data input processing on the Raman spectral data for learning 37L. The data input processing is processing for selectively leaving only data (intensity value) in a specific spectral region in the Raman spectral data for learning 37L to obtain the post-processing Raman spectral data for learning 37LP. Selectively leaving only the data in the specific spectral region means, in other words, selectively deleting data other than the data in the specific spectral region.

The specific spectral region is three regions of a first region, a second region, and a third region described below. First, the first region is a region having a wave number of 470 cm⁻¹ to 550 cm⁻¹ (470 cm⁻¹ or more and 550 cm⁻¹ or less). The second region is a region having a wave number of 860 cm⁻¹ to 900 cm⁻¹ (860 cm⁻¹ or more and 900 cm⁻¹ or less). The third region is a region having a wave number of 1060 cm⁻¹ to 1120 cm⁻¹ (1060 cm⁻¹ or more and 1120 cm⁻¹ or less).

The first region is a region attributed to the disulfide bond DB of the antibody 16. The second region is a region attributed to the aromatic amino acid of the antibody 16. The third region is a region attributed to a carbon-carbon bond, a carbon-nitrogen bond, and a carbon-oxygen bond of the antibody 16.

The learning unit 68 inputs the post-processing Raman spectral data for learning 37LP to the prediction model 61 and outputs the prediction result for learning 75L from the prediction model 61. Next, the learning unit 68 compares the prediction result for learning 75L with the correct fragment content 75CA and performs the loss calculation of the prediction model 61 using the loss function based on the comparison result. The learning unit 68 performs update setting of the prediction model 61 according to a result of the loss calculation and updates the prediction model 61 according to the update setting.

The learning unit 68 repeatedly performs the series of processing for the data input processing, the input of the post-processing Raman spectral data for learning 37LP to the prediction model 61, the output of the prediction result for learning 75L from the prediction model 61, the loss calculation, the update setting, and the update of the prediction model 61 while changing the training data 80. The learning unit 68 ends the repetition of the series of processing in a case where the prediction accuracy of the prediction result for learning 75L with respect to the correct fragment content 75CA reaches a predetermined set level. The learning unit 68 outputs the prediction model 61 of which the prediction accuracy reaches the set level in this way to the RW control unit 66 as the trained prediction model 61. The learning may be ended in a case where the series of processing is repeated a set number of times regardless of the prediction accuracy of the prediction result for learning 75L with respect to the correct fragment content 75CA. In addition, the learning of the prediction model 61 may be continued even after being stored in the storage 50.

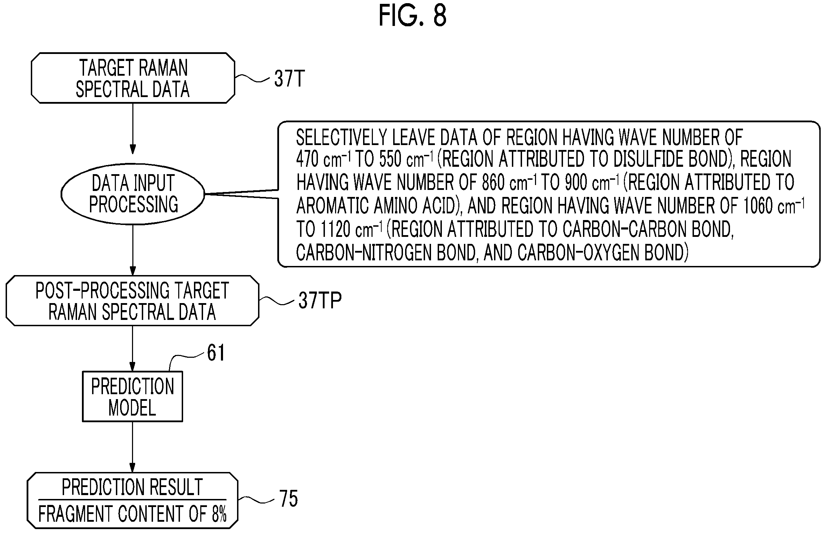

As shown in FIG. 8 as an example, the prediction unit 69 performs the same data input processing as in the case of the learning unit 68 on the target Raman spectral data 37T to obtain the post-processing target Raman spectral data 37TP. The prediction unit 69 inputs the post-processing target Raman spectral data 37TP to the prediction model 61 and outputs the prediction result 75 from the prediction model 61. The prediction result 75 includes a predicted value of the fragment content.

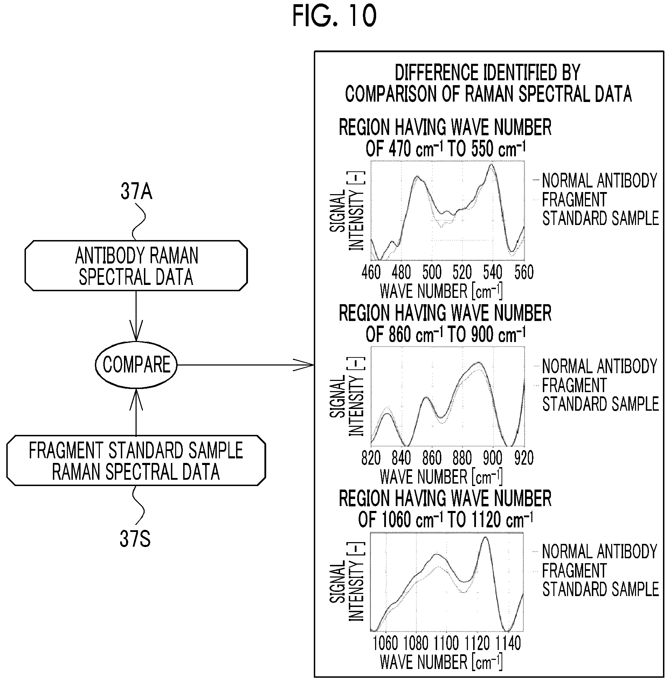

Here, how the specific spectral region was found will be described with reference to FIGS. 9 and 10.

First, as shown in FIG. 9 as an example, a standard sample (hereinafter, referred to as a fragment standard sample) 90 of the fragment 18 is artificially generated from the antibody 16. Then, fragment standard sample Raman spectral data 37S (see FIG. 10) which is the Raman spectral data 37 of the fragment standard sample 90 is obtained. The fragment standard sample Raman spectral data 37S is an example of "spectral data of a fragment" and "spectral data of a standard sample of a fragment" according to the technology of the present disclosure.

More specifically, the CHO cells are cultured in the culture unit 14 to produce the antibody 16, and the culture supernatant liquid 17 is obtained (Step ST10). Next, the culture supernatant liquid 17 is passed through the immunoaffinity chromatography device 20 to obtain a first purified liquid 23 (Step ST11).

A reducing agent 91 such as TCEP is added to the first purified liquid 23, and the first purified liquid 23 is left to stand in an environment of 37°C for 1 hour to cause a reduction reaction in the antibody 16 in the first purified liquid 23 (Step ST12). Then, it is confirmed by gel electrophoresis that the antibody 16 in the first purified liquid 23 is completely converted into the fragment 18 (Step ST13).

The reducing agent 91 is preferably the exemplified TCEP, but is not limited thereto. For example, dithiothreitol, 2-mercaptoethanol, tributylphosphine, 3-mercapto-1,2-propanediol, glutathione, cysteine, or the like may be used as the reducing agent 91. In addition, the antibody 16 may be converted into the fragment 18 by an enzyme reaction instead of the reduction reaction. Further, the antibody 16 may be converted into the fragment 18 by changing the temperature and/or the hydrogen ion exponent of the first purified liquid 23.

The first purified liquid 23 after the reduction reaction is subjected to ultrafiltration and diafiltration using the filter 28 to remove the reducing agent 91 (Step ST14). In this way, the fragment standard sample 90 is obtained. The reducing agent 91 may be removed from the first purified liquid 23 using a chromatography device.

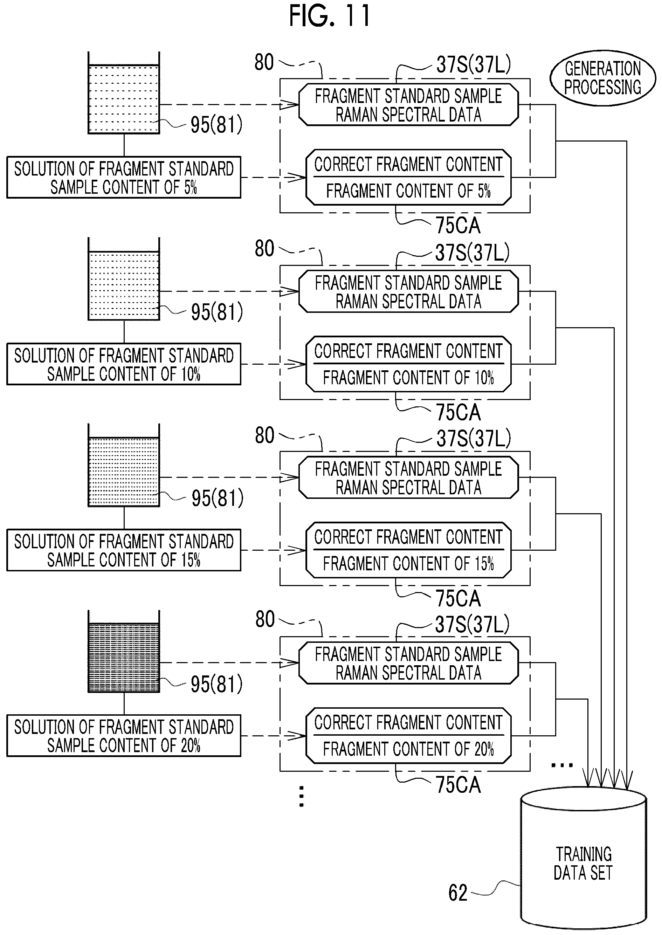

Next, a fragment standard sample solution 95 (see FIG. 11) in which only the obtained fragment standard sample 90 is dispersed as a component in a liquid is produced. Then, the Raman scattered light RSL emitted from the fragment standard sample solution 95 is measured by the Raman spectrometer 12 to obtain the fragment standard sample Raman spectral data 37S (Step ST15). It is ideal that only the fragment standard sample 90 is dispersed in the fragment standard sample solution 95, but a substance other than the fragment standard sample 90 may be mixed in the fragment standard sample solution 95 in an amount that is generally allowed in the technical field to which the disclosed technology belongs and that does not contradict the gist of the disclosed technology.

On the other hand, the ultrafiltration and the diafiltration in Step ST14 are performed on the first purified liquid 23 obtained in Step ST11 without performing the reduction reaction in Step ST12 and the confirmation of the fragmentation by gel electrophoresis in Step ST13. In this way, the antibody 16 is obtained. Next, an antibody solution in which only the obtained antibody 16 is dispersed as a component in a liquid is produced. Then, the Raman scattered light RSL emitted from the antibody solution is measured by the Raman spectrometer 12 to obtain the antibody Raman spectral data 37A (see FIG. 10) which is the Raman spectral data 37 of the antibody 16. The antibody Raman spectral data 37A is an example of "spectral data of a biological molecule" according to the disclosed technology. It is ideal that only the antibody 16 is dispersed in the antibody solution as in the fragment standard sample solution 95, but a substance other than the antibody 16 may be mixed in the antibody solution in an amount that is generally allowed in the technical field to which the disclosed technology belongs and that does not contradict the gist of the disclosed technology.

As shown in FIG. 10 as an example, in a case where the antibody Raman spectral data 37A and the fragment standard sample Raman spectral data 37S are compared, it is found that there is a clear difference in intensity values with high reproducibility in a first region having the wave number of 470 cm⁻¹ to 550 cm⁻¹, a second region having the wave number of 860 cm⁻¹ to 900 cm⁻¹, and a third region having the wave number of 1060 cm⁻¹ to 1120 cm⁻¹. Therefore, the first to third regions are defined as the specific spectral regions.

In addition, as shown in FIG. 11 as an example, in the disclosed technology, the user U produces a plurality of fragment standard sample solutions 95 having different fragment contents as one type of the reference suspension 81 by using the fragment standard samples 90 generated by the procedures of Steps ST10 to ST14 in FIG. 9. Then, the Raman scattered light RSL emitted from each of the plurality of fragment standard sample solutions 95 is measured by the Raman spectrometer 12 to obtain a plurality of pieces of fragment standard sample Raman spectral data 37S. A set of the fragment standard sample Raman spectral data 37S obtained in this way and the fragment content of each fragment standard sample solution 95 is stored in the training data set 62 as the training data 80. That is, the fragment standard sample Raman spectral data 37S of the fragment standard sample 90 artificially generated from the antibody 16 is included in the training data 80 as the Raman spectral data for learning 37L. The processing shown in FIG. 11 is an example of "generation processing" according to the disclosed technology.

In a case where the instruction to start the operation program 60 by the user U is received at the instruction receiving unit 67, the display control unit 70 performs control to display a data input screen 100 shown in FIG. 12 as an example on the display 41. The data input screen 100 is provided with an input box 101 for the target Raman spectral data 37T and a prediction button 102. A file of the target Raman spectral data 37T can be dragged and dropped into the input box 101. Dragging and dropping the file of the target Raman spectral data 37T into the input box 101 is an instruction to input the target Raman spectral data 37T of the second purified liquid 25 of which the fragment content is to be predicted.

The user U inputs the desired target Raman spectral data 37T into the input box 101 and then selects the prediction button 102. In a case where the prediction button 102 is selected, the instruction receiving unit 67 receives an instruction to predict the fragment content related to the target Raman spectral data 37T input to the input box 101. As a result, the processing shown in FIG. 8 is executed in the prediction unit 69, and the prediction result 75 is output from the prediction unit 69 to the display control unit 70.

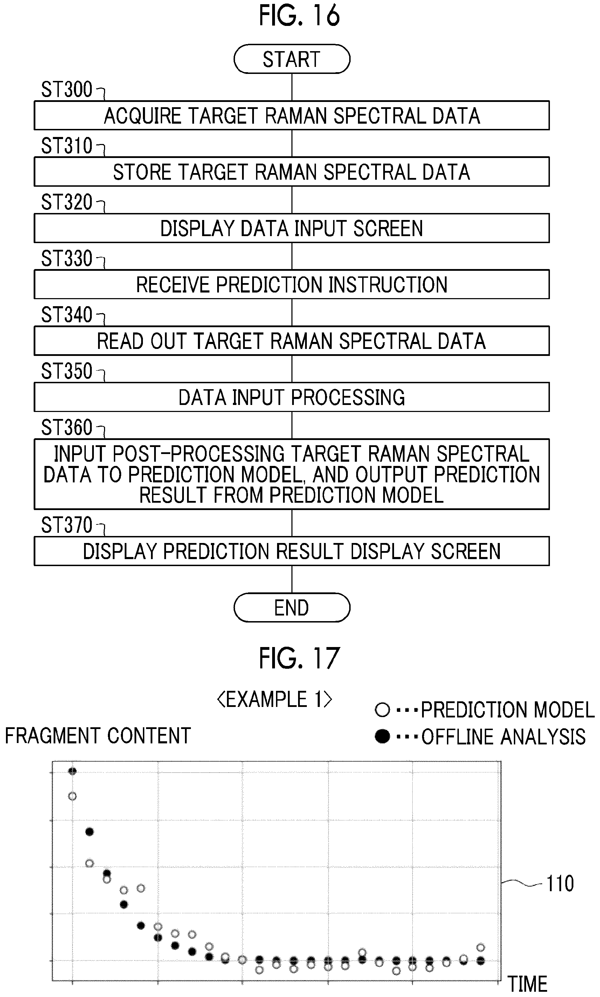

The display control unit 70 receives the prediction result 75 from the prediction unit 69 and performs control to display a prediction result display screen 105 shown in FIG. 13 as an example on the display 41. The fragment content, which is the prediction result 75, is displayed on the prediction result display screen 105. In addition, a save button 106 and an OK button 107 are provided at a lower portion of the prediction result display screen 105. In a case where the save button 106 is selected, the prediction result 75 is stored in the storage 50 in association with the target Raman spectral data 37T. In a case where the OK button 107 is selected, the display of the prediction result display screen 105 is erased. For example, the fragment content may be predicted by the prediction model 61 at a plurality of time points at regular intervals, the fragment content at the plurality of time points may be obtained, the fragment content at the plurality of time points may be plotted on a graph in which the lateral axis is time, and the temporal transition of the fragment content may be displayed on the prediction result display screen 105 (see FIG. 17).

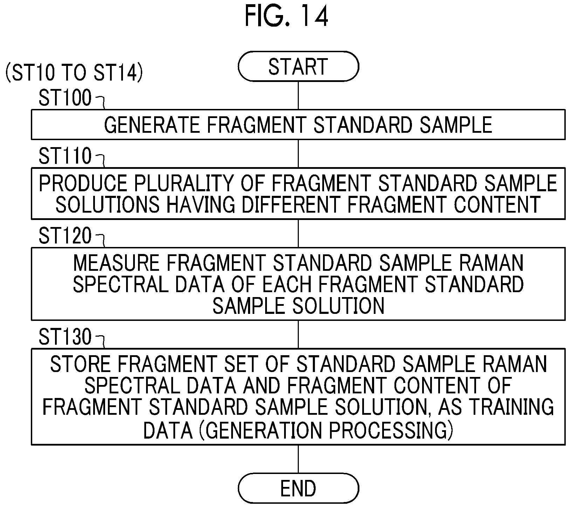

Next, the operation of the above configuration will be described with reference to the flowcharts shown in FIGS. 14, 15, and 16 as an example. As shown in FIG. 6, the CPU 52 of the information processing apparatus 40 functions as the acquisition unit 65, the RW control unit 66, the instruction receiving unit 67, the learning unit 68, the prediction unit 69, and the display control unit 70 by starting the operation program 60.

First, as shown in FIG. 14, the fragment standard sample 90 is generated through the procedures of steps ST10 to ST14 shown in FIG. 9 (step ST100). Using the fragment standard sample 90, a plurality of fragment standard sample solutions 95 having different fragment contents are produced as shown in FIG. 11 (step ST110). Next, the fragment standard sample Raman spectral data 37S of each fragment standard sample solution 95 is measured (step ST120). Then, a set of the fragment standard sample Raman spectral data 37S and the fragment content of the fragment standard sample solution 95 is stored in the training data set 62 as the training data 80 (step ST130). As a result, the training data set 62 includes the training data 80 composed of a set of the Raman spectral data for learning 37L of various reference suspensions 81 and the correct fragment content 75CA, and also includes the training data 80 composed of a set of the fragment standard sample Raman spectral data 37S of the fragment standard sample solution 95 and the fragment content thereof.

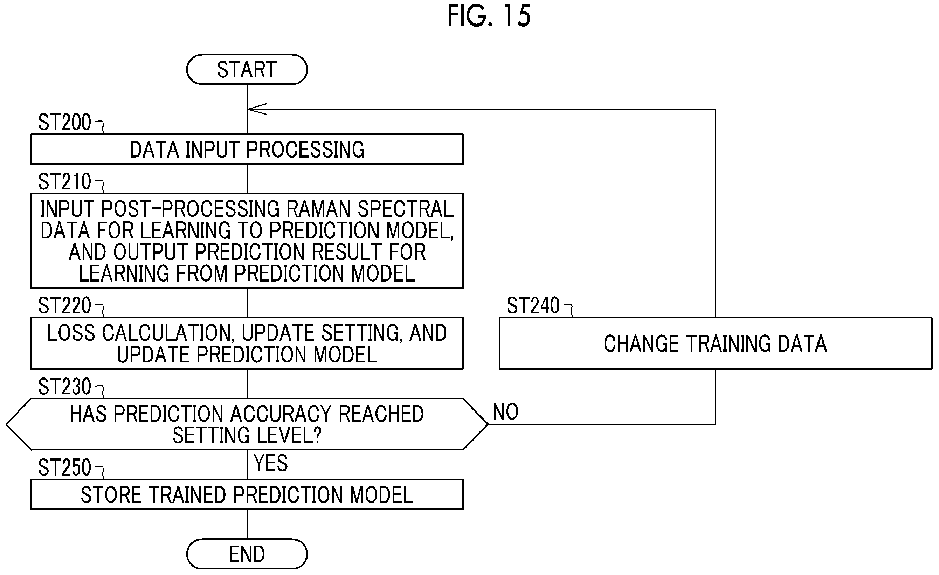

As shown in FIG. 15, in the training phase of the prediction model 61, the learning unit 68 performs the data input processing on the Raman spectral data for learning 37L as shown in FIG. 7 (step ST200). The data input processing is processing for selectively leaving the data in the specific spectral region in the Raman spectral data for learning 37L to obtain the post-processing Raman spectral data for learning 37LP.

Subsequently, the post-processing Raman spectral data for learning 37LP is input to the prediction model 61, and the prediction result for learning 75L is output from the prediction model 61 (step ST210). The prediction result for learning 75L and the correct fragment content 75CA are compared with each other, and the loss calculation of the prediction model 61 using the loss function is performed based on the comparison result. Then, the update setting of the prediction model 61 is performed according to the result of the loss calculation, and the prediction model 61 is updated according to the update setting (step ST220).

A series of processing for steps ST200 to ST220 is repeatedly performed while the prediction accuracy of the prediction result for learning 75L with respect to the correct fragment content 75CA does not reach a predetermined set level (NO in step ST230), and the training data 80 is changed (step ST240). In a case where the prediction accuracy of the prediction result for learning 75L with respect to the correct fragment content 75CA reaches the predetermined set level (YES in step ST230), the series of processing for steps ST200 to ST220 is ended. The trained prediction model 61 is output from the learning unit 68 to the RW control unit 66, and is stored in the storage 50 under the control of the RW control unit 66 (step ST250).

As shown in FIG. 16, the acquisition unit 65 acquires the target Raman spectral data 37T from the Raman spectrometer 12 (step ST300). The target Raman spectral data 37T is output from the acquisition unit 65 to the RW control unit 66, and is stored in the storage 50 under the control of the RW control unit 66 (step ST310).

The data input screen 100 shown in FIG. 12 is displayed on the display 41 under the control of the display control unit 70 (step ST320). In a case where the user U inputs the desired target Raman spectral data 37T into the input box 101 and selects the prediction button 102 on the data input screen 100, the prediction instruction of the fragment content related to the target Raman spectral data 37T input to the input box 101 is received at the instruction receiving unit 67 (step ST330). The prediction instruction is output from the instruction receiving unit 67 to the RW control unit 66.

The target Raman spectral data 37T for which the input instruction and the prediction instruction are issued is read out from the storage 50 under the control of the RW control unit 66 (step ST340). In addition, the prediction model 61 is also read out from the storage 50. The target Raman spectral data 37T and the prediction model 61 are output from the RW control unit 66 to the prediction unit 69.

In the prediction unit 69, as shown in FIG. 8, the data input processing is executed on the target Raman spectral data 37T (step ST350). The data input processing is processing for selectively leaving the data in the specific spectral region of the target Raman spectral data 37T to obtain the post-processing target Raman spectral data 37TP.

Subsequently, the post-processing target Raman spectral data 37TP is input to the prediction model 61, and the prediction result 75 is output from the prediction model 61 (step ST360). The prediction result 75 is output from the prediction unit 69 to the display control unit 70.

As shown in FIG. 13, the prediction result display screen 105 is displayed on the display 41 under the control of the display control unit 70 (step ST370), and the fragment content of the prediction result 75 is provided for the viewing of the user U.

The user U makes various decisions according to the prediction result 75 displayed on the prediction result display screen 105. For example, a case where the conditions such as the culture conditions of the antibody-producing cells are set by a small-scale facility is considered. In this case, in a case where the prediction result 75 is worse than the target value, the user U makes a decision to stop the current experiment and to proceed to an experiment under new conditions. In addition, a case where the condition setting is completed and mass production is performed by a large-scale facility is considered. In this case, in a case where the prediction result 75 is worse than the target value, the user U makes a decision to interrupt the mass production and to perform the maintenance of the culture tank of the culture unit 14 or the immunoaffinity chromatography device 20 and/or the cation exchange chromatography device 21 of the purification unit 15.

As described above, the information processing apparatus 40 uses the prediction model 61. In the prediction model 61, the generation processing and the data input processing according to the difference occurring in the specific spectral region between the antibody Raman spectral data 37A and the fragment standard sample Raman spectral data 37S are performed. In addition, the information processing apparatus 40 comprises the acquisition unit 65 and the prediction unit 69. The acquisition unit 65 acquires the target Raman spectral data 37T of the second purified liquid 25 in which the amount of the fragment 18 is unknown. The prediction unit 69 predicts the fragment content by inputting the target Raman spectral data 37T to the prediction model 61. By performing the generation processing and the data input processing according to the difference occurring in the specific spectral region between the antibody Raman spectral data 37A and the fragment standard sample Raman spectral data 37S on the prediction model 61, it is possible to accurately predict the fragment content in the second purified liquid 25 in which the antibody 16 and the fragment 18 are mixed, as compared with a case where no measures are taken.

As shown in FIG. 8, the data input processing is processing for selectively inputting the data of the specific spectral region of the target Raman spectral data 37T to the prediction model 61. In addition, as shown in FIG. 7, the data input processing is processing for selectively inputting the data of the specific spectral region of the Raman spectral data for learning 37L to the prediction model 61.

The specific spectral region is a region in which a difference occurs between the antibody Raman spectral data 37A and the fragment standard sample Raman spectral data 37S. Therefore, the data of the specific spectral region is data representing a slight structural difference between the antibody 16 and the fragment standard sample 90, and further between the antibody 16 and the fragment 18. Therefore, it can be said that the data of the specific spectral region is data that greatly contributes to the prediction of the fragment content of the second purified liquid 25 in which the antibody 16 and the fragment 18 are mixed. In other words, it can be said that the data other than the specific spectral region is data that does not contribute much to the improvement of the accuracy of the prediction of the fragment content of the second purified liquid 25 in which the antibody 16 and the fragment 18 are mixed, and may be noise. Therefore, in a case where the data input processing is processing for selectively inputting the data of the specific spectral region to the prediction model 61, the prediction accuracy of the fragment content by the prediction model 61 can be further improved.

As shown in FIG. 7, the prediction model 61 is generated by using the training data 80 including the Raman spectral data for learning 37L which is the Raman spectral data 37 of the reference suspension 81 in which the amount of the fragment 18 is known. Then, as shown in FIG. 11, the generation processing is processing for including the fragment standard sample Raman spectral data 37S which is the Raman spectral data 37 of the fragment standard sample 90 artificially generated from the antibody 16, as the Raman spectral data for learning 37L in the training data 80.

The fragment standard sample Raman spectral data 37S is data that is purely affected only by the fragment standard sample 90, in other words, data that is not affected by the antibody 16 at all. Therefore, by including the fragment standard sample Raman spectral data 37S in which the influence of the antibody 16 that may be noise in the learning of the prediction model 61 is eliminated, in the training data 80, the learning of the prediction model 61 is further promoted, and as a result, the prediction accuracy of the fragment content by the prediction model 61 can be further improved. In addition, since the fragment standard sample solution 95 having any fragment content can be freely produced, and a large amount of the fragment standard sample Raman spectral data 37S can be obtained, it is possible to greatly contribute to the enrichment of the training data 80.

In the present embodiment, an example in which both the generation processing and the data input processing are performed has been described, but the present disclosure is not limited thereto. It is sufficient that at least one of the generation processing or the data input processing is performed.

The biopharmaceutical including the antibody 16 is called an antibody drug and is widely used not only for the treatment of chronic diseases, such as cancer, diabetes, and rheumatoid arthritis, but also for the treatment of rare diseases, such as hemophilia and a Crohn's disease. Therefore, according to the present example in which the biological molecule is a protein and the protein is the antibody 16, it is possible to promote the development of antibody drugs that are widely used for the treatment of various diseases.

The Raman scattered light RSL is likely to reflect information derived from a functional group of an amino acid of a protein. Therefore, by using the electromagnetic wave as the Raman scattered light RSL as in the present example, it is possible to obtain the Raman spectral data 37 that clearly reflects the physical properties such as the fragment content of the antibody 16 which is a protein.

As shown in FIGS. 7, 8, and 10, the specific spectral region is a region attributed to the disulfide bond DB of the protein, a region attributed to the aromatic amino acid, and a region attributed to the carbon-carbon bond, the carbon-nitrogen bond, and the carbon-oxygen bond. More specifically, the specific spectral region is a region having a wave number of 470 cm⁻¹ to 550 cm⁻¹, a region having a wave number of 860 cm⁻¹ to 900 cm⁻¹, and a region having a wave number of 1060 cm⁻¹ to 1120 cm⁻¹. Therefore, it can be said that the specific spectral region is appropriate in a case where the biological molecule is a protein.

The specific spectral region may be a region attributed to at least any one of the disulfide bond DB of the protein, the aromatic amino acid, the carbon-carbon bond, the carbon-nitrogen bond, or the carbon-oxygen bond. Similarly, the specific spectral region may be at least one of a region having a wave number of 470 cm⁻¹ to 550 cm⁻¹, a region having a wave number of 860 cm⁻¹ to 900 cm⁻¹, or a region having a wave number of 1060 cm⁻¹ to 1120 cm⁻¹.

The specific spectral region is more preferably a region attributed to the carbon-carbon bond, the carbon-nitrogen bond, and the carbon-oxygen bond. That is, the specific spectral region is more preferably a region having a wave number of 1060 cm⁻¹ to 1120 cm⁻¹.

As shown in FIG. 2, the fragment 18 is the heavy chain HC, the light chain LC, the fragment antigen-binding region FabR, and the fragment crystallizable region FcR of the antibody 16. These are general as the fragment 18 of the antibody 16. Therefore, the fragment content of the general fragment 18 can be accurately predicted.

The fragment 18 may be at least one of the heavy chain HC, the light chain LC, the fragment antigen-binding region FabR, or the fragment crystallizable region FcR of the antibody 16.

As shown in FIG. 1, the target suspension is the second purified liquid 25 obtained by passing the culture supernatant liquid 17 of CHO cells which are antibody-producing cells through the immunoaffinity chromatography device 20 and the cation exchange chromatography device 21. The immunoaffinity chromatography device 20 and the cation exchange chromatography device 21 play a role of capturing the fragment 18. Therefore, in a case where the target suspension is the second purified liquid 25, it is possible to monitor whether or not the immunoaffinity chromatography device 20 and the cation exchange chromatography device 21 play a role. In a case where it is determined that the immunoaffinity chromatography device 20 and/or the cation exchange chromatography device 21 do not play a role based on the prediction result 75, an appropriate response such as maintenance of the immunoaffinity chromatography device 20 and/or the cation exchange chromatography device 21 can be taken.

As shown in FIG. 10, the specific spectral region is a region identified by comparing the antibody Raman spectral data 37A with the fragment standard sample Raman spectral data 37S. In the related art, it was considered that the information on the molecular structures of the antibody 16 and the fragment 18 does not change, and there is no difference between the antibody Raman spectral data 37A and the fragment standard sample Raman spectral data 37S. However, the fragment standard sample 90 was artificially generated from the antibody 16 again, and when the antibody Raman spectral data 37A and the fragment standard sample Raman spectral data 37S were compared in detail, it was found that there is a difference in data in the specific spectral region. The technology of the present disclosure actually utilizes the valuable knowledge of the specific spectral region identified in this way to improve the prediction accuracy of the fragment content by the prediction model 61.

As shown in FIG. 6, the prediction model 61 is a machine learning model. The machine learning model is generally used to predict unknown parameters, and the prediction accuracy can be increased to a certain level through learning. Therefore, the prediction model 61 having a relatively high prediction accuracy can be easily generated.

As shown in FIG. 1, the concentration of the fragment 18 in the second purified liquid 25 is 0.1 g/L to 5.0 g/L. As described above, the technology of the present disclosure can accurately predict the fragment content even in a case where the concentration of the fragment 18 is relatively low.

### [Examples]

First, as Example 1, the prediction model 61 that had been trained by performing the generation processing shown in FIG. 11 and the data input processing shown in FIG. 7 was prepared. Then, as shown in FIG. 1, the target Raman spectral data 37T of the second purified liquid 25 was measured by the measurement system 10. The target Raman spectral data 37T was measured a plurality of times at regular time intervals from the start of the operation of the cation exchange chromatography device 21 (the start of the generation of the second purified liquid 25). Then, the data input processing shown in FIG. 8 was performed on the target Raman spectral data 37T, and the prediction model 61 was caused to predict the fragment content.

On the other hand, the second purified liquid 25 from which the target Raman spectral data 37T was measured was recovered each time using the fraction collector. Then, the recovered second purified liquid 25 was subjected to offline analysis by an HPLC device or the like, the amount of the fragment 18 in the recovered second purified liquid 25 was actually measured, and the fragment content was calculated based on the measured amount.

The temporal transitions of the fragment content predicted by the prediction model 61 and the fragment content obtained by the offline analysis in Example 1 are shown in the graph 110 of FIG. 17. According to the graph 110, it was found that both the temporal transitions of the fragment content predicted by the prediction model 61 and the fragment content obtained by the offline analysis had the same tendency. As a result, it was confirmed that the prediction of the fragment content by the prediction model 61 was successfully performed.

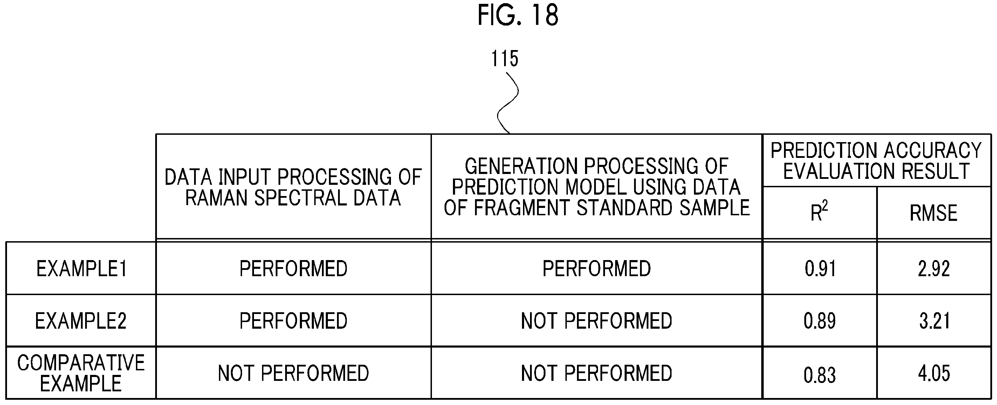

In addition, a coefficient of determination R² of the fragment content predicted by the prediction model 61 with respect to the fragment content obtained by the offline analysis and a root mean squared error (RMSE) were derived, and a degree of deviation of the fragment content predicted by the prediction model 61 with respect to the fragment content obtained by the offline analysis, that is, the prediction accuracy of the fragment content predicted by the prediction model 61 was evaluated. The results are shown in Table 115 of FIG. 18. The coefficient of determination R² in Example 1 was 0.91, and the root mean squared error RMSE was 2.92. According to these numerical values, it can be said that Example 1 has a very high prediction accuracy.

Example 2 is a case in which the data input processing shown in FIGS. 7 and 8 was performed, but the generation processing shown in FIG. 11 was not performed. The coefficient of determination R² in Example 2 was 0.89, and the root mean squared error RMSE was 3.21. According to these numerical values, it can be said that Example 2 also has a high prediction accuracy, although it is inferior to Example 1.

### [Comparative Example]

The comparative example is a case in which the data input processing shown in FIGS. 7 and 8 and the generation processing shown in FIG. 11 were not performed. That is, the comparative example may be rephrased as the related art example. The coefficient of determination R² in the comparative example was 0.83, and the root mean squared error RMSE was 4.05.

In both Example 1 and Example 2, the coefficient of determination R² and the root mean squared error RMSE are improved as compared with Comparative Example 1. Therefore, it was found that, in a case in which at least the data input processing was performed, the prediction accuracy of the fragment content by the prediction model 61 could be improved as compared with the related art. This result can be said to support that the data in the specific spectral region is effective for improving the prediction accuracy of the fragment content.

In addition, in Example 1, the coefficient of determination R² and the root mean squared error RMSE are improved as compared with Example 2. Therefore, it was found that, in a case in which both the data input processing and the generation processing were performed, the prediction accuracy of the fragment content by the prediction model 61 could be improved as compared with a case in which only the data input processing was performed. From the above, according to the technology of the present disclosure, it was confirmed that it is possible to accurately predict the fragment content in the second purified liquid 25 in which the antibody 16 and the fragment 18 are mixed.

The fragment content is predicted as the amount of the fragment 18, but the present disclosure is not limited thereto. The amount of the fragment 18 itself, the concentration of the fragment 18, or the like may be predicted. The amounts of two or more kinds of the fragments 18, such as the fragment content and the concentration, may be predicted.

The antibody 16 may be a bispecific antibody, an antibody-drug conjugate, a low-molecular-weight antibody, a glycan-modified antibody, or the like.

The protein is not limited to the antibody 16. The biological molecule may be a peptide, a nucleic acid (DNA or ribonucleic acid (RNA)), a lipid, a virus, a virus subunit, a virus-like particle, or the like. The protein may be a cytokine (interferon, interleukin, or the like), a hormone (insulin, glucagon, follicle-stimulating hormone, erythropoietin, or the like), a growth factor (insulin-like growth factor (IGF)-1, basic fibroblast growth factor (bFGF), or the like), a blood coagulation factor (a seventh factor, an eighth factor, a ninth factor, or the like), an enzyme (a lysosomal enzyme, a deoxyribonucleic acid (DNA) degrading enzyme, or the like), a fragment crystallizable (Fc) fusion protein, a receptor, albumin, or a protein vaccine. Therefore, the fragment 18 is not limited to the heavy chain HC, the light chain LC, or the like of the antibody 16 described as an example.

The electromagnetic wave is not limited to the Raman scattered light RSL, and thus the spectral data is not limited to the Raman spectral data 37. The spectral data may be infrared absorption spectral data, near infrared absorption spectral data, nuclear magnetic resonance spectral data, ultraviolet visible absorption spectroscopy (UV-Vis) spectral data, or fluorescence spectral data.

The prediction model 61 is not limited to the machine learning model. A model generated by a multivariate analysis or a statistical analysis may be used. Examples of the multivariate analysis and the statistical analysis include linear regression, multiple regression, principal component regression, partial least squares regression, logistic regression, Lasso regression, ridge regression, support vector regression, and Gaussian process regression. Among these, the principal component regression is more preferable. In the model generated by such multivariate analysis or statistical analysis, determining the coefficient of the regression equation based on at least two pieces of the training data 80 is an example of "generated using calibration data" according to the technology of the present disclosure. In addition, the prediction model 61 may be a rule-based model.

The target suspension is not limited to the second purified liquid 25 described as an example. The target suspension may be the first purified liquid 23 or the third purified liquid 26. In addition, the target suspension may be the cell culture solution before the cell removal by the cell removal filter in the culture unit 14, or may be the culture supernatant liquid 17 after the cell removal.

In the above-described embodiment, so-called in-line sensing has been described as an example, but the present disclosure is not limited thereto. The technology of the present disclosure may be applied to offline sensing.

The information processing apparatus 40 may be a personal computer that is installed in a pharmaceutical facility as illustrated in FIG. 1 or may be a server computer that is installed in a data center independent of the pharmaceutical facility.

In a case where the information processing apparatus 40 is configured by a server computer, the target Raman spectral data 37T is transmitted from a personal computer installed in each pharmaceutical facility to the server computer via a network such as the Internet. The server computer distributes various screens such as a data input screen 100 and a prediction result display screen 105 to the personal computer in a format of screen data for web distribution created by a markup language such as extensible markup language (XML). The personal computer reproduces a screen displayed on a web browser based on the screen data and displays the reproduced screen on the display. Another data description language such as JavaScript (registered trademark) Object Notation (JSON) may be used instead of XML.

A hardware configuration of the computer constituting the information processing apparatus 40 according to the disclosed technology can be variously modified. For example, the information processing apparatus 40 may be configured by a plurality of computers separated as hardware in order to improve processing capacity and reliability. For example, the functions of the acquisition unit 65, the prediction unit 69, and the display control unit 70 and the function of the learning unit 68 are distributed to two computers. In this case, the information processing apparatus 40 is composed of two computers.

The hardware configuration of the computer of the information processing apparatus 40 can be appropriately changed in accordance with required performance such as processing capacity, safety, and reliability. Further, not only the hardware but also an application program, such as the operation program 60, may be duplicated or may be dispersively stored in a plurality of storages in order to ensure safety and reliability.

In the above-described embodiment, for example, as a hardware structure of a processing unit that executes various types of processing, such as the acquisition unit 65, the RW control unit 66, the instruction receiving unit 67, the learning unit 68, the prediction unit 69, and the display control unit 70, various processors shown below can be used. As described above, the various processors include, in addition to the CPU 52 that is a general-purpose processor executing software (operation program 60) to function as various processing units, a programmable logic device (PLD) that is a processor of which a circuit configuration can be changed after manufacture, such as a field programmable gate array (FPGA), and a dedicated electrical circuit that is a processor having a circuit configuration dedicatedly designed to execute specific processing, such as an application specific integrated circuit (ASIC).

One processing unit may be configured by one of the various processors, or may be configured by a combination of two or more processors of the same type or different types (for example, a combination of a plurality of FPGAs and/or a combination of a CPU and an FPGA). In addition, a plurality of processing units may be configured by one processor.

As an example in which the plurality of processing units are configured by one processor, first, as represented by a computer such as a client and a server, there is a form in which one processor is configured by a combination of one or more CPUs and software and the processor functions as the plurality of processing units. Second, as represented by a system on chip (SoC) or the like, there is a form in which a processor that realizes the functions of the entire system including a plurality of processing units by one integrated circuit (IC) chip is used. In this manner, various processing units are configured by using one or more of the various processors as a hardware structure.

Further, as a hardware structure of the various processors, more specifically, an electrical circuit (circuitry) in which circuit elements such as semiconductor elements are combined can be used.

The technology according to the following supplementary notes can be understood from the above description.

### [Supplementary Note 1]

An information processing apparatus that predicts an amount of a fragment of a biological molecule based on spectral data obtained by measuring electromagnetic waves emitted from a suspension in which the biological molecule and the fragment of the biological molecule are dispersed as components in a liquid, the information processing apparatus comprising:
a processor,
in which the processor is configured to:
   use a prediction model in which at least one of generation processing or data input processing according to a difference occurring in a specific spectral region between spectral data of the biological molecule and spectral data of the fragment is executed;
   acquire target spectral data of a target suspension in which the amount of the fragment is unknown; and
   input the target spectral data to the prediction model to predict the amount of the fragment.

### [Supplementary Note 2]

The information processing apparatus according to Supplementary Note 1, in which the data input processing is processing for selectively inputting data of the specific spectral region among the target spectral data to the prediction model.

### [Supplementary Note 3]

The information processing apparatus according to Supplementary Note 1 or 2, in which the prediction model is generated by using calibration data including reference spectral data of a reference suspension in which the amount of the fragment is known, and
the generation processing is processing for including spectral data of a standard sample of the fragment artificially generated from the biological molecule in the calibration data as the reference spectral data.

### [Supplementary Note 4]

The information processing apparatus according to Supplementary Note 3, in which the data input processing is processing for selectively inputting data of the specific spectral region among the reference spectral data to the prediction model.

### [Supplementary Note 5]

The information processing apparatus according to any one of Supplementary Notes 1 to 4, in which the biological molecule is a protein, and the electromagnetic waves are Raman scattered light.

### [Supplementary Note 6]

The information processing apparatus according to Supplementary Note 5, in which the specific spectral region is a region attributed to at least one of a disulfide bond, an aromatic amino acid, a carbon-carbon bond, a carbon-nitrogen bond, or a carbon-oxygen bond of the protein.

### [Supplementary Note 7]

The information processing apparatus according to Supplementary Note 6, in which the specific spectral region is at least one of a region having a wave number of 470 cm⁻¹ to 550 cm⁻¹, a region having a wave number of 860 cm⁻¹ to 900 cm⁻¹, or a region having a wave number of 1060 cm⁻¹ to 1120 cm⁻¹.

### [Supplementary Note 8]

The information processing apparatus according to any one of Supplementary Notes 5 to 7, in which the protein is an antibody.

### [Supplementary Note 9]

The information processing apparatus according to Supplementary Note 8, in which the fragment is at least one of a heavy chain, a light chain, a fragment antigen-binding region, and a fragment crystallizable region of the antibody.

### [Supplementary Note 10]

The information processing apparatus according to Supplementary Note 8 or 9, in which the target suspension is a purified liquid obtained by passing a culture supernatant liquid of a cell that produces the antibody through a chromatography device.

### [Supplementary Note 11]

The information processing apparatus according to any one of Supplementary Notes 1 to 10, in which the specific spectral region is a region identified by comparing the spectral data of the biological molecule with spectral data of a standard sample of the fragment artificially generated from the biological molecule.

### [Supplementary Note 12]

The information processing apparatus according to any one of Supplementary Notes 1 to 11, in which the prediction model is a machine learning model.

### [Supplementary Note 13]

The information processing apparatus according to any one of Supplementary Notes 1 to 12, in which a concentration of the fragment in the target suspension is 0.1 g/L to 5.0 g/L.

The technology of the present disclosure can also appropriately combine various embodiments and/or various modification examples described above. The technology of the present disclosure is not limited to the above embodiment and may adopt various configurations without departing from its gist. Furthermore, the technology of the present disclosure extends to a storage medium that non-transitorily stores the program, and a computer program product including the program, in addition to the program.

The description and drawings set forth above are detailed descriptions of parts related to the technology of the present disclosure and are merely an example of the technology of the present disclosure. For example, the above description of the configuration, the function, the action, and the effect is a description of examples of the configuration, the function, the action, and the effect of the parts according to the technology of the present disclosure. Accordingly, it goes without saying that unnecessary parts may be deleted, new elements may be added, or replacements may be made with respect to the description content and the shown content described above within a range that does not depart from the gist of the technology of the present disclosure. In order to avoid complications and facilitate understanding of the portions related to the disclosed technology, in the contents described and shown above, common technical knowledge that does not particularly require description is not described in order to enable the implementation of the disclosed technology.

In the present specification, "A and/or B" is synonymous with "at least one of A or B". That is, "A and/or B" means that it may be only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated in the present specification by reference to the same extent as in a case where each of the documents, the patent applications, and the technical standards are specifically and individually indicated to be incorporated by reference.

## Claims

1. An information processing apparatus that predicts an amount of a fragment of a biological molecule based on spectral data obtained by measuring electromagnetic waves emitted from a suspension in which the biological molecule and the fragment of the biological molecule are dispersed as components in a liquid, the information processing apparatus comprising:
a processor,
wherein the processor is configured to:
use a prediction model in which at least one of generation processing or data input processing according to a difference occurring in a specific spectral region between spectral data of the biological molecule and spectral data of the fragment is executed;
acquire target spectral data of a target suspension in which the amount of the fragment is unknown; and
input the target spectral data to the prediction model to predict the amount of the fragment.

2. The information processing apparatus according to claim 1, wherein the data input processing is processing for selectively inputting data of the specific spectral region among the target spectral data to the prediction model.

3. The information processing apparatus according to claim 1, wherein the prediction model is generated by using calibration data including reference spectral data of a reference suspension in which the amount of the fragment is known, and
the generation processing is processing for including spectral data of a standard sample of the fragment artificially generated from the biological molecule in the calibration data as the reference spectral data.

4. The information processing apparatus according to claim 3, wherein the data input processing is processing for selectively inputting data of the specific spectral region among the reference spectral data to the prediction model.

5. The information processing apparatus according to claim 1, wherein the biological molecule is a protein, and the electromagnetic waves are Raman scattered light.

6. The information processing apparatus according to claim 5, wherein the specific spectral region is a region attributed to at least one of a disulfide bond, an aromatic amino acid, a carbon-carbon bond, a carbon-nitrogen bond, or a carbon-oxygen bond of the protein.

7. The information processing apparatus according to claim 6, wherein the specific spectral region is at least one of a region having a wave number of 470 cm⁻¹ to 550 cm⁻¹, a region having a wave number of 860 cm⁻¹ to 900 cm⁻¹, or a region having a wave number of 1060 cm⁻¹ to 1120 cm⁻¹.

8. The information processing apparatus according to claim 5, wherein the protein is an antibody.

9. The information processing apparatus according to claim 8, wherein the fragment is at least one of a heavy chain, a light chain, a fragment antigen-binding region, and a fragment crystallizable region of the antibody.

10. The information processing apparatus according to claim 8, wherein the target suspension is a purified liquid obtained by passing a culture supernatant liquid of a cell that produces the antibody through a chromatography device.

11. The information processing apparatus according to claim 1, wherein the specific spectral region is a region identified by comparing the spectral data of the biological molecule with spectral data of a standard sample of the fragment artificially generated from the biological molecule.

12. The information processing apparatus according to claim 1, wherein the prediction model is a machine learning model.

13. The information processing apparatus according to claim 1, wherein a concentration of the fragment in the target suspension is 0.1 g/L to 5.0 g/L.

14. An operation method for an information processing apparatus that predicts an amount of a fragment of a biological molecule based on spectral data obtained by measuring electromagnetic waves emitted from a suspension in which the biological molecule and the fragment of the biological molecule are dispersed as components in a liquid, the operation method for an information processing apparatus comprising:
using a prediction model in which at least one of generation processing or data input processing according to a difference occurring in a specific spectral region between spectral data of the biological molecule and spectral data of the fragment is executed;
acquiring target spectral data of a target suspension in which the amount of the fragment is unknown; and
inputting the target spectral data to the prediction model to predict the amount of the fragment.

15. An operation program of an information processing apparatus that predicts an amount of a fragment of a biological molecule based on spectral data obtained by measuring electromagnetic waves emitted from a suspension in which the biological molecule and the fragment of the biological molecule are dispersed as components in a liquid, the operation program causing a computer to execute a process comprising:
using a prediction model in which at least one of generation processing or data input processing according to a difference occurring in a specific spectral region between spectral data of the biological molecule and spectral data of the fragment is executed;
acquiring target spectral data of a target suspension in which the amount of the fragment is unknown; and
inputting the target spectral data to the prediction model to predict the amount of the fragment.
